Europäisches Patentamt

European Patent Office

Office européen des brevets

(11) Publication number: **0 038 642**
**B1**

(12) **EUROPEAN PATENT SPECIFICATION**

(45) Date of publication of patent specification: **08.02.89**

(21) Application number: **81301470.1**

(22) Date of filing: **03.04.81**

(60) Divisional application **87103532 filed on 11.03.87.**

(51) Int. Cl.⁴: **C 12 P 1/00, C 07 K 1/00, A 61 K 39/42, G 01 N 33/50, C 12 N 7/02, C 12 N 5/00, C 12 N 15/00 // C12R1/91**

(54) **A monoclonal antibody against hepatitis B and its production, a hybridoma producing the monoclonal antibody and compositions containing it, the propagation of the hybridoma and the monoclonal antibody for use as an antiviral agent against hepatitus B and in isolating hepatitus b surface antigen from a sample.**

(30) Priority: **09.04.80 GB 8011697**

(43) Date of publication of application:
**28.10.81 Bulletin 81/43**

(45) Publication of the grant of the patent:
**08.02.89 Bulletin 89/06**

(84) Designated Contracting States:
**BE CH DE FR GB IT LI NL SE**

(56) References cited:
**GB-A-2 000 186**

**TRANSFUSION (Philadelphia), vol. 19, no. 5, September/October 1979, Philadelphia, US J.W.K. SHIH et al. "Production of monoclonal antibodies against Hepatitis B surface antigen by somatic cell hybrids" page 637.**

**GASTROENTEROLOGY, vol. 77, no. 5, 1979 J.R. WANDS et al. "Production and characterization of monoclonal antibodies to Hepatitis B surface antigen (HBs Ag) by cellular hybridization", page A46.**

(73) Proprietor: **NATIONAL RESEARCH DEVELOPMENT CORPORATION**
**101 Newington Causeway**
**London SE1 6BU (GB)**

(72) Inventor: **Goodall, Alison Helena**
**Eastview Hare Street Great Hormead**
**Buntingford Hertfordshire (GB)**
Inventor: **Janossy, George**
**24 Twyford Road**
**Harrow Middlesex (GB)**
Inventor: **Thomas, Howard Christopher**
**103 Oakwood Road**
**London NW11 (GB)**

(74) Representative: **Goldin, Douglas Michael et al J.A. KEMP & CO. 14, South Square Gray's Inn London WC1R 5EU (GB)**

(56) References cited:
**FEDERATION PROCEEDINGS, vol. 39, no. 3, march 1, 1980. W.A. PRESENT et al. "Characterization of murine hybridoma antibodies to Hepatitis B surface antigenic determinants", page 929, point 3484.**

Courier Press, Leamington Spa, England.

# EP 0 038 642 B1

(56) References cited:
GASTROENTEROLOGY, vol. 79, no. 5 part 2, November 1980. J.R. WANDS et al. "Immunodiagnosis of Hepatitis B by high affinity monoclonal anti-HB antibodies", page 1063

GASTROENTEROLOGY, vol. 79, no. 5, part 2, November 1980, J.R. WANDS et al. "Identification of epitopes on HBs Ag polypeptides by analysis with monoclonal anti-HBs antibodies", page 1063.

**Description**

The present invention relates to a monoclonal antibody to hepatitis B surface antigen (HBsAg) which is secreted by a new hybridoma cell line, and particularly the use of this monoclonal antibody in assay systems for hepatitis B virus infections.

The development of new cell lines that can be continuously subcultured has been an important area of research in recent years. One particular application for such cell lines lies in the production of antibodies, and the introduction of somatic cell hybridisation (i.e. cell fusion) has provided a research tool which enables production of pure, monoclonal antibodies. By fusing antibody secreting cells with myeloma cells and cloning the resulting antibody-secreting hybrids in tissue culture, it has been found possible to produce and select a stable hybrid monoclonal cell line that is capable of secreting a particular antibody. It is thus possible to ensure the production of pure, monospecific antibodies since, in the monoclonal cell line, they arise from one original antibody-secreting cell.

This ability to produce pure, monospecific antibodies is clearly of great utility for accurate screening, purification of the antigen and in therapeutic/preventative applications and the present invention is directed towards the use of these techniques in connection with hepatitis B virus infections.

The diagnosis of acute and chronic hepatitis B infection is dependent upon the detection of HBs antigen in a patient's blood. This has generally been achieved by a radioimmunoassay utilising rabbit or horse anti HBs serum absorbed onto polystyrene beads. The HBs antigen in the patient's serum binds to the solid phase antibody and can then be detected by a further application of radiolabelled anti-HBs. Another technique for detecting HBs-Ag in the liver employs double-layer immunofluorescence. These techniques do, however, involve a primary procedure where the antiserum is raised in rabbits and the antibodies purified by expensive and time-consuming affinity chromatography.

The use of radioimmunoassay techniques for detecting HBs antigen is well established in connection with blood transfusion services but has not been extensively implemented for screening hospital patients in general. Apart from the obvious desirablity of this for reducing risk of infection within the hospital, the identification in particular of obstetric patients who are carriers of hepatitis virus can be of great importance since identification of the carrier mother may allow administration of hyperimmune globulin to the infant shortly after birth and thus considerably reduce the risk of its developing hepatitis and of becoming a chronic carrier.

Any increase in the screening for heptatis B virus infection and in the possible treatment of the disease will demand large quantities of readily available, high quality anti-HBs serum. A new hybrid monoclonal cell line has now been found and designated RF-HBs-1, which has been shown to be stable in culture and secretes a monoclonal antibody to hepatitis B surface antigen. Moreover, this cell line is capable of storage in, and recovery from, liquid nitrogen and thus provides a readily available supply of pure monospecific antibody to hepatitis B surface antigen.

The present invention accordingly provides a monoclonal antibody to hepatitis B surface antigen which is secreted by the hybridoma cell line RF-HBs-1. Desirably, the monoclonal antibody is in substantially pure form free from other immunological material.

The monoclonal (and hence monospecific) antibody of the present invention may be recovered from a culture supernatant of from mouse ascites fluid or serum as is indicated hereinafter. The monoclonal antibody of the invention shows activity against HBs-Ag and is precipitated by rabbit anti-serum raised against mouse IgG$_1$ heavy chain and against mouse K light chain but not by rabbit anti-serum to mouse IgG$_{2a}$, IgG$_{2a}$, IgM or IgA or to mouse λ light chains. Thus, the monoclonal antibody is an IgG$_{1(K)}$ mouse immunoglobin that reacts with major subtypes of the HBsAg (ad and ay) and can be produced by culturing the RF-HBs-1 cell line.

The cell line of the invention RF-HBs-1, is a hybridoma cell line which has been produced by fusing spleen cells from Balb/c mice, previously immunized with concentrated hepatitis B surface antigen (HBsAGg), with the HAT-sensitive mouse myeloma cell line (P3-NS1/1-Ag4-1), according to the methods of Kohler and Milstein (Eur J Immunol 6, 292 (1976)). By cloning procedures using the fused cell line, the hybridoma monoclonal cell line designated RF-HBs-1 has been isolated.

The cell line RF-HBs-1 appears as a fairly uniform population of cells which morphologically resemble the parent NS1 myeloma in both size and shape having a mean size of 13 μm. They each appear spherical in culture and have a centrally located nucleus with a large cytoplasmic area, equal to or greater than the nuclear volume. RF-HBs-1 differs from the parent line in that although, like the parental line, it grows partially attached to a solid substratum, e.g. glass or plastic, it can be detached from this surface by vigorous agitation and does not require trypsinisation or treatment with EGTA. It also grows as a suspension culture in stirrer culture vessels. The cells have doubling time of 14.6 hours and will grow in RPMI-FCS medium (see below) each requiring feeding at 2—3 day intervals and sub-culturing at 2—3 day intervals.

The hybridoma cell line RF-HBs-1 secretes a monoclonal antibody and the cell line has continued to secrete antibody for at least three months in continuous culture.

The present invention also provides the hybridoma cell line designated RF-HBs-1 which secretes monoclonal antibodies to hepatitis B surface antigen, the cell line preferably being in substantially pure form free from other cellular material, with a nutrient medium capable of maintaining the cell line. An appropriate medium for this cell line contains a source of carbon, a source of nitrogen and if desired, vitamins, and/or inorganic salts, and an example of such a medium is RPMI-1640 supplemented with foetal calf serum e.g. RPMI-FCS (see below).

In the preparation of the hybridoma cell line RF-HBs-1, Balb/c mice were immunised by intraperitoneal injection of 0.2 ml of purified HBs antigen diluted 1:1 with complete Freunds adjuvant (CFA) at a final antigen concentration of 0.7 mg/ml. (The HBS antigen (subtype adw) used was purified from HBs antigen positive serum by caesium chloride density gradient centrifugation). Four weeks later, the mice were boosted with a further 0.2 ml injection of the HBs antigen/CFA mixture. Six weeks later the mouse that gave the highest serum titre of antibody to HBS antigen, as determined by solid phase radioimmunoassay, was boosted with a further 0.2 ml I.P. injection of HBs antigen in CFA, and killed 5 days after this final boost and the spleen removed.

The spleen was teased in RPMI-1640 medium, supplemented with 10% v/v foetal calf serum, 2m M glutamine and 100 IU each of penicillin and streptomycin (RPMI-FCS) at 37°C, pH 7.2 and sodium bicarbonate concentration of 2g/l, and the resultant suspension was passed several times, gently, in and out of a sterile 10 ml syringe. The single cell suspension was decanted from the few residual lumps of unseparated spleen and the cells were washed twice in serum-free RPMI-1640, centrifuging at 1500 g for 5 minutes. The washed cell pellet was resuspended in RPMI-1640.

A semi-confluent culture of mouse myeloma cells $P_3$-NSI/I-Ag4-1 (i.e. a culture of cells in an active stage of growth having been maintained in continuous culture at 37°C in RPMI-1640 medium supplemented with 10% (v/v) foetal calf serum, 2 mM glutamine, 100 IU of penicillin and 100 µg streptomycin and $2 \times 10^{-5}$ M 8-thioguanine buffered with bicarbonate/$CO_2$) were detached from their culture vessel by mild trypsinisation and washed twice in serum-free RPMI-1640 by centrifugation at 400 g. The washed cells were resuspended in RPMI-1640 and counted.

In order to effect hybridisation, the total spleen cell population was mixed with the myeloma cells in a round-bottomed, sterile, 10 ml centrifuge tube in a ratio of 10 to 1 spleen cells to myeloma cells, the co-pelleted by centrifugation at 1500 g. After removal of the supernatant, 1 ml of a 40% (w/w) solution of polyethylene glycol (PEG) 1500 in RPMI-1640, that had been prewarmed to 37°C, was added to the cell pellet and gently mixed with the cells. The cell-PEG mixture was incubated at 37°C for exactly 7 minutes and then the PEG solution was gradually diluted off the cells by repeated addition of equal volumes of RPMI-1640, until the PEG concentration was below 0.5% (w/w). The suspension was centrifuged at 1500 g and the cell pellet gently resuspended and washed twice in serum-free RPMI-1640, centrifuging at 1000 g. The washed cell pellet was gently resuspended in 1 ml of RPMI-FCS, and incubated for two hours at 37°C and the cells were then distributed, at $1 \times 10^6$ cells/ml in RPMI-FCS, into $24 \times 2$ ml multiwell tissue culture plates and cultured at 37°C in a 5% $CO_2$/95% air atmosphere.

Twenty-four hours after fusion, half of the culture medium in each flask was replaced by HAT medium (RPMI-FCS supplemented with $1 \times 10^{-4}$M hypoxanthine, $4 \times 10^{-7}$M aminopterin and $1.6 \times 10^{-5}$M thymidine). The cultures were supplemented with fresh HAT medium, in this way, every day for the first 3 days and at 2—3 day intervals thereafter, to ensure selection of hybrid cells with the ability to grow in HAT medium.

The HAT medium was replaced by HT medium (RPMI-FCS + hypoxanthine and thymidine) in stages, beginning at day 40 after fusion and then adapted to growth in RPMI-FCS at day 52 after fusion.

Supernatants from the hybrid cultures were assayed for antibody to HBs antigen as soon as the wells showed growth of more than 100 cells. Only one culture well contained antibody-producing cells though other culture wells did contain viable hybrid cells.

The antibody producing hybrid cells from this one culture well were then cloned, initially by limiting dilution of the original cultures onto a feeder layer of spleen cells from a non-immunised Balb/c mouse, growing the hybrids in $96 \times 0.2$ ml cluster dishes in RPMI-FCS. Growth was produced in 16 wells that had been confirmed as containing only one hydrid cell at the start of the cloning schedule, and from these the cell line RF-HBs-1 was selected as providing a stable hybrid cell line secreting monoclonal antibodies to HBs antigen. This clone of cells has subsequently been recloned twice with the result of 100% antibody-producing clones.

A further aspect of the present invention resides in a process for the propagation of the hybridoma cell line RF-HBs-1 which comprises culturing the cells of this cell line in a nutrient culture medium therefor. This method of propagation also represents a means of producing the antibody of the invention which may be separated from the culture medium. An appropriate nutrient culture medium for this cell line contains a source of nitrogen and, if desired, vitamins and/or inorganic salts, and is, for example, RPMI-1640, supplemented with foetal calf serum e.g. in an amount of about 10% by weight based on the culture medium. RPMI-FCS described above is particularly suitable. When growing the cells, a suitable initial concentration is $1 \times 10^5$ cells/ml for RF-HBs-1. These concentrations should desirably not be allowed to exceed $1 \times 10^6$ cells/ml.

In addition to culturing this cell line in a nutrient medium, it is also possible to implant it into a mouse to establish an ascites tumour; this is generally carried out by intraperitoneal injection of, for example, $4 \times 10^5$—$1 \times 10^6$ cells per mouse, into pristane (2,6,10,14-tetramethyl pentadecane)-primed, Balb/c mice. Establishment of an ascites tumour may also be achieving in non-pristane-primed mice in which case, when propagating RF-HBs-1 cells $1 \times 10^6$—$2 \times 10^6$ cells per mouse should be injected. The cell lines may also be grown as a solid tumour by subcutaneous injection.

The monoclonal antibodies secreted by the hybridoma may then be recovered from the ascites fluid or from the mouse serum and this process of both propagating the cell line and producing the antibody in respect of cell line RF-HBs-1 represents a still further aspect of the present invention. This means of obtaining the monoclonal antibody does offer the advantage that the yield of antibody may, for example, be as much as 10-fold higher than the yield obtainable from the bulk culture of the cells. It will normally take about 2 of weeks for the cells to grow up in the mice.

The antibody may be used unpurified from such sources as described above, or, preferably, are subjected to purification before use. For example, antibodies to be coupled to sepharose for use in affinity chromatography are generally purified by ammonium sulphate precipitation, while antibodies to be iodinated or biotin-conjugated are generally subjected to Protein A-purification.

The hybridoma cells may be stored in FCS-supplemented RPMI-1640 medium containing say 20% FCS by freezing in liquid nitrogen at, for example, $5 \times 10^6$ cells/ml or higher using 10% dimethylsulphoxide as cryoprotectant. The freezing rate is preferably controlled between +40°C and −100°C e.g. at 1—2°C/minute and the frozen cells can be stored below −100°C e.g. at −170°C in a liquid nitrogen refrigerator.

The monospecific antibody produced by cell line RF-HBs-1 is of particular value in providing accurate screening tests for patients infected by hepatitis B virus. This antibody can be used in assay systems, employing for example, immunofluorescent microscopy or immuno-electron microscopy, in detecting the presence of HBsAg in cellular material or in serum. Quantitative assays may be carried out by solid-phase radiometric assay, and RF-HBs-1 antibody is useful in this respect since it recognises an antigenic epitope that appears with relatively high frequency on the HBsAg molecule. However, particularly useful solid-phase radiometric assay systems employ combinations of RF-HBs-1 with other monoclonal antibodies.

Alternative assay methods, in accordance with the invention, may, instead of employing a radiolabel, employ e.g. an enzyme-label or a biotin-label which will generally be linked to the RF-HBs-1 antibody. Further, it is also possible to use the RF-HBs-1 antibody of the invention in a haemagglutination assay for HBs-Ag.

The monoclonal antibody of this invention coupled to a solid-phase such as cyanogen bromide-activated Sepharose 4B (CnBr-S4B; Pharmacia Fine Chemicals, Uppsala, Sweden), can also be used to remove HBs-Ag e.g. from human or animal material, either for the purpose of preparing HBs-Ag in a purified form for use in preparing vaccines or for the removal of HBs-Ag from material to be given patients. Thus, the present invention also provides a method of isolating hepatitis B surface antigen from a biological sample which comprises contacting the biological sample with a solid phase comprising RF-HBs-1 antibody to bring about binding of antigen to antibody and subsequently separating the desired purified material from the solid phase.

The RF-HBs-1 monoclonal antibody is also of value in clinical applications as an anti-viral agent where antibody is to be administered directly to patients. The monoclonal antibody may have a role in the treatment of patients carrying the heptatis B virus and may neutralised the infectivity of the virus by inhibiting its combination with the receptor site on cells and also enhance the phagocytosis and intracellular digestion of the viruses. Potentially the use of the monoclonal antibody in this context as a substitute for hyper-immune gamma globulin may avoid giving large amounts of non-specific immunoglobulin along with the small amounts of the specific antibody, which is the case with conventional serum. The RF-HBs-1 antibody, may also serve to break tolerance in HBV carriers by binding to the HBs-Ag and making it more immunogenic.

The monoclonal cell line RF-HBs-1 has been deposisted with the collection Nationale de Cultures de Microorganismes (C.N.C.M.) at the Institut Pasteur, Paris on 26 March, 1980 and has been given the accession number I-117.

The present invention will now be further illustrated by the following Examples.

## Example 1

In Vitro Culturing of Cell Line RF-HBs-1

RF-HBs-1 cells were seeded at $1 \times 10^5$ cells/ml in RPMI-1640 medium supplemented with between 5 and 20% (v/v) foetal calf serum. The growth rate of the cells was essentially the same in all media (doubling time of 14.6 hours ±2.5) but growth was maintained for a longer period of time in 15 and 20% foetal calf serum.

A seeding density of $1 \times 10^2$ cells/ml is recommended for optimal growth although lower seeding densities can be employed but result in a longer lag phase of growth. Subculturing should be effected every 2—3 days.

## Example 2

In Vivo Culturing of Cell Line RF-HBs-1

20 six week old Balb/c mice were primed with an intraperitoneal injection of 0.5 ml pristane (2,6,10,14-tetramethyl-pentadecane) at day 1 and day 7. At day 14 each mouse was implanted intraperitoneally with 1 $\times 10^5$ viable hybridoma RF-HBs-1 cells. 10 days following implantation all the mice were showing signs of massive tumour growth and were sacrificed, and the ascites fluid collected via a 19 gauge needle. The ascites fluid was centrifuged at 2000 g and the clear supernatants containing RF-HBs-1 antibody collected and stored at −20°C.

### Example 3
#### Coating of Polystyrene Beads with RF-HBs-1 Monoclonal Antibodies

Polystyrene beads were carried with the monoclonal antibody in the form of ascitic fluid at various dilutions in pH 9.6 bicarbonate buffer (0.015M $Na_2CO_3$:0.035M $NaHCO_3$) by incubating for 1 hour, at 20°C with agitation, followed by at least 16 hours incubation at 4°C in the same antibody-buffer mixtures. The beads were then washed and incubated for 4 hours, at room temperature, with 200 µl $^{125}$I-HBs-Ag (Abbott Laboratories) to determine the optimum coating conditions (see Table 1 below).

### TABLE 1

| Dilution of ascitic fluid | RF-HBs-1 Antibody |
|---|---|
| 1:10 | 12,500 |
| 1:25 | - |
| 1:50 | 15,100 |
| 1:100 | 17,900 |
| 1:200 | - |
| 1:400 | - |
| 1:1000 | 6090 |

The pH of 9.6 for the coating buffer used in this Example has been found to be optimal.

The beads, once coated with antibody can be dried, following 3 washes with distilled water, by centrifugation over an absorbant pad. These beads will remain stable at −20°C for at least 3 months.

### Example 4
#### Recognition of HBs-Ag

The monoclonal antibody produced by the cell line RF-HBs-1 recognises an epitope that is common to the ad and ay subtypes of HBsAg.

Polystyrene beads coated with HBsag of mixed subtype (AUSAB: Abbott Laboratories) were incubated overnight at room temperature with 50 µg/ml of either Protein A-purified RF-HBs-1 antibody, or with an equivalent titre of horse antiserum to HBs-Ag, and then for a further four hours at room temperature with $^{125}$I-RF-HBs-1 or [125] antibody I, iodinated as described in Example 5 (100,000 cpm). The results are given in Table 2 below.

### TABLE 2

| Blocking antibody | Percentage Binding $^{125}$I-RF-HBs-1 Antibody |
|---|---|
| 50µg/ml RF-HBs-1 | 52% |
| Horse anti-serum to HBsAg (at equivalent titre) | 70% |
| Control (50% NBCS) | 100% |

The results show that RF-HBs-1 antibody inhibits its own binding. Horse anti-serum did, on the other hand, inhibit RF-HBs-1 antibody binding.

## Example 5

### Use of Cell Line Antibodies in Radiometric assay

RF-HBs-1 antibody was purified from ascitic fluid from mice implanted with the RF-HBs-1 cell line by elution at pH 5.5 from a protein A-Sepharose column (Pharmacia Fine Chemicals, Uppsala, Sweden) according to the method of Prowse & Jenkin (Immunochemistry *15* 429—436, 1978). 5 µg of RF-HBs-1 antibody purified in this way, were iodinated with 0.5 mCi $^{125}$I using 2.5 µg Chloramine T in a total reaction volume of 15 µl, incubating for 30 seconds at 4°C, so as to give an equimolar binding ratio of $^{125}$I to antibody.

Polystyrene beads coated with RF-HBs-1 antibody or antibodies, or Ausria II beads, were incubated with 200 µl of either HBsAG −ve human serum or with 200 µl of human serum containing 20 ng/ml HBsAG, for 2 hours, at 45°, washed and incubated for a further 1 hour at 45° with $^{125}$I-RF-HBs-1 antibody, (approx 100,000 cpm per assay), washed and counted. Results are expressed either as cpm (mean of at least 2 samples) or as the ratio between the counts obtained with the HBsAG +ve sample and the HBsAG −ve sample (+/−).

TABLE 3

| Solid phase antibody | HBsAg | Tracer antibody | | | |
|---|---|---|---|---|---|
| | | RF-HBs-1 | | AUSRIA II | |
| | | ·CPM | +/- | CPM | +/- |
| RF-HBs-1 | + | 694 | 21 | 1723 | 27 |
| | - | 33 | | 64 | |
| AUSRIA II | + | 1393 | 19.6 | 3445 | 59 |
| | - | 71 | | 58 | |

## Example 6

### Limits of Detection of HBsAg

Polystyrene beads, coated with the optimal concentration of RF-HBs-1 antibody were incubated for 2 hours, at 45°C, with 200 µl of either HBsAg −ve human serum or with 200 µl of human serum containing known amounts of HBsAg, washed and incubated with 200 µl $^{125}$I-RF-HBs-1 (200,000 cpm per assay) for further one hour at 45°C.

The results are given in Table 4 below which also gives results of a similar titration of HBsAg using an AUSRIA II RIA. Results are expressed both as cpm (mean of at least 2 experiments) or as +/− ratios.

TABLE 4

| HBsAg (ng/ml) | Tracer antibody | | | AUSRIA II | |
|---|---|---|---|---|---|
| | Solid-phase antibody | RF-HBs-1 | | AUSRIA II | |
| | | cpm | +/- | cpm | +/- |
| 20 | | 510 | 7.3 | 4197 | 35 |
| 10 | | 207 | 3.0 | 1188 | 9.8 |
| 5 | | 149 | 1.7 | 500 | 4.1 |
| 2.5 | | 87 | 1.2 | 230 | 1.9 |
| 1.25 | | 62 | 1.0 | - | - |
| 0.6 | | - | - | - | - |
| 0.3 | | - | - | 150 | 1.2 |
| 0.15 | | - | - | - | - |
| 0.075 | | - | - | - | - |
| 0 | | 61 | | 121 | - |

Example 7

Use of RF-HBs-1 antibody in affinity chromatography of HBsAg

RF-HBs-1 antibody, partially purified from ascitic fluid by ammonium sulfate precipitation, was coupled to CNBr-S4B (Pharmacia Fine Chemicals, Uppsala, Sweden) according to the manufacturers instruction. 100 ml of human serum containing HBsAg were passed down this column, resulting in a 98.7% removal of the HBsAg from the serum. In addition, 100% of the removed antigen could be eluted from the column using 3M KI. The antigen was immunogenic in mice and rabbits and was shown to be free from other human serum contaminants by immunoelectrophoresis against a rabbit antiserum to whole serum.

Example 8

Use of RF-HBs-1 antibody in the localisation of HBsAg in cellular material

RF-HBs-1 antibody in supernatants from cultures of RF-HBs-1 cells was used to identify HBsAg in frozen sections of liver biopsies from HBV-bearing humans or chimpanzees or in cells bearing the virus, visualizing the antigen-antibody complex with a goat antiserum to mouse immunoglobulin, coupled to a fluorescent molecule. Furthermore, Protein A-purified RF-HBs-1 antibody was conjugated to biotin by incubation of 1 mg of RF-HBs-1 antibody in 1 ml of pH 8.3 bicarbonate-saline buffer (0.1M $NaHCO_3$:0.5M NaCl) for 4 hours, at 20°C, with 90 µg biotin succinamide dissolved at 1 mg/ml in DMSO. Following an overnight dialysis at 4°C, against phosphate-buffered saline, the RF-HBs-1 antibody-biotin conjugate was used, in combination with an avidin-fluorochrome conjugate or with avidin-ferritin, to visualise HBsAg by immunofluorescent microscopy and by immuno-electron microscopy, respectively.

**Claims**

1. A monoclonal antibody to hepatitis B surface antigen which is secreted by hybridoma cell line RF-HBs-1 (C.N.C.M. I-117).

2. An antibody according to claim 1 substantially free from other immunological material.

3. A process of producing a monoclonal antibody to hepatitis B surface antigen which comprises culturing hybridoma cell line RF-HBs-1 (C.N.C.M. I-117) and recovering the antibody produced.

4. A process according to claim 3 in which the cell line is cultured *in vitro* in a nutrient culture medium therefor and the antibody is recovered from culture supernatant.

5. A process according to claim 3 in which the cell line is established as an ascites tumour in a mouse by implanting the cell line, and the antibody is recovered from ascites fluid or serum of the mouse.

6. A method of isolating hepatitis B surface antigen from a biological sample, characterised by contacting the biological sample with a solid phase comprising RF-HBs-1 antibody so that the hepatitis B surface antigen binds to the antibody and subsequently separating the hepatitis B surface antigen from the solid phase.

7. Hybridoma cell line RF-HBs-1 (C.N.C.M. I-117) which secretes a monoclonal antibody to hepatitis B surface antigen.

8. The cell line RF-HBs-1 according to claim 7 substantially free from other cellular material.

9. A process of propagating a hybridoma cell line as claimed in claim 7 or 8 *in vitro* which comprises culturing the cell line in a nutrient culture medium therefor.

10. A process of propagating a hybridoma cell line as claimed in claim 7 or 8 *in vitro* which comprises implanting the cell line in a mouse to establish an ascites tumour.

11. A composition comprising a hybridoma cell line as claimed in claim 7 or 8 and a nutrient medium capable of maintaining the cell line.

12. A monoclonal antibody secreted by hybridoma cell line RF-HBs-1 (C.N.C.M. I-117) for use as an anti-viral agent against hepatitis B virus.

**Patentansprüche**

1. Monoklonaler Antikörper gegen Hepatitis B-Oberflächenantigen, der von einer Hybridom-Zellinie RF-HBs-1 (C.N.C.M. I-117) produziert wird.

2. Antikörper nach Anspruch 1, der im wesentlichen von anderem immunologischen Material frei ist.

3. Verfahren zur Herstellung eines monoklonalen Antikörpers gegen Hapatitis B-Oberflächenantigen durch Kultivierung der Hybridom-Zellinie RF-HBs-1 (C.N.C.M. I-117) und Gewinnung des produzierten Antikörpers.

4. Verfahren nach Anspruch 3 durch Kultivierung der Zellinie *in vitro* in einem Nähr-Kulturmedium hierführ und Gewinnung des Antikörper aus der obersten Kulturschicht.

5. Verfahren nach Anspruch 3, wobei die Zellinie als Aszites-Tumor in einer Maus durch Implantation der Zellinie etabliert und der Antikörper aus der Aszites-Flüssigkeit oder dem Serum der Maus gewonnen wird.

6. Verfahren zur Isolierung von Hepatitis B-Oberflächenantigen aus einer biologischen Probe, dadurch gekennzeichnet, daß die bioloische Probe mit einer RF-HBs-1 Antikörper enthaltenden, festen Phase in Kontakt gebracht wird, so daß sich das Hepatitis B-Oberflächenantigen an den Antikörper bindet, und anschließend das Hepatitis B-Oberflächenantigen aus der festen Phase abgetrennt wird.

7. Hydridom-Zellinei RF-HBs-1 (C.N.C.M. I-117), welche einen monoklonalen Antikörper gegen Hepatitis B-Oberflächenantigen produziert.

8. Zellinie RF-HBs-1 nach Anspruch 7, die im wesentlichen von anderem Zellmaterial frei ist.

9. Verfahren zur Vermehrung einer Hydridom Zellinie nach Anspruch 7 oder 8 *in vitro* durch Kultivierung der Zellinie in einem Nähr-Kulturmedium hierfür.

10. Verfahren zur Vermehrung einer Hydridom Zellinie nach Anspruch 7 oder 8 *in vitro* durch Implantieren der Zellinie in einer Maus zur Erzeugung von einem Aszites-Tumor

11. Zusammensetzung enthaltend eine Hybridom-Zellinie nach Anspruch 7 oder 8 und ein die Zellinie erhaltendes Nährmedium.

12. Ein von einer Hybridom-Zellinie RF-HBs-1 (C.N.C.M. I-117) produzierter Antikörper zur Verwendung als anti-virales Agens gegen Hepatitis B Virus.

**Revendications**

1. Anticorps monoclonal contre l'antigène de surface de l'hépatite B qui est sécréte par la lignèe cellulaire d'hybridome RF-HBs-1(C.N.C.M. I-117).

2. Anticorps selon la revendicatión 1, sensiblement exempt d'autres substances immunologiques.

3. Procédé de production d'un anticorps monoclonal contre l'antigène de surface de l'hépatite B dans lequel on cultive la lignée cellulaire d'hybridome RF-HBs-1 (C.N.C.M. I-117) et on recueille l'anticorps produit.

4. Procédé selon la revendication 3, dans lequel on cultive la lignée cellulaire *in vitro* dans un milieu de culture nutritif appropriée, on recueille l'anticorps à partir du surnageant de culture.

5. Procédé selon la revendication 3, dans lequel la lignée cellulaire est établie sous la forme d'une tumeur ascitique chez une souris par implantation de la lignée cellulaire, et l'anticorps est recuelli à partir du fluide ascitique ou du sérum de la souris.

6. Procédé d'isolement de l'antigène de surface de l'hépatite B à partir d'un échantillon biologique, caractérisé en ce qu'on met en contact l'échantillon biologique avec un phase solide comprenant l'anticorps RF-HBs-1 de manière que l'antigène de surface de l'hépatite B se lie à l'anticorps puis en ce qu'on sépare l'antigène de surface de l'hépatite B de la phase solide.

7. Lignée cellulaire d'hydribrome RF-HBs-1 (C.N.C.M. I-117) qui sécrète un anticorps monoclonal contre l'antigène de surface de l'hépatite B.

8. Lignée cellulaire RF-HBs-1 selon la revendication 7, sensiblement exempte d'autre matériaux cellulaires.

9. Procédé de propagation d'un lignée cellulaire d'hybridome telle que revendiquée dans la revendication 7 ou 8 *in vitro,* dans lequel on cultive la lignée cellulaire dans un milieu de culture nutritif approprié.

10. Procédé de propagation d'un lignée cellulaire d'hybridome telle que revendiquée dans la revendication 7 ou 8 *in vitro,* dans lequel on implante la lignée cellulaire chez une souris pour établir une tumeur ascitique.

11. Composition comprenant une lignée cellulaire d'hybridome telle que revendiquée dans la revendication 7 ou 8 et un milieu nutritif capable d'entretenir la lignée cellulaire.

12. Anticorps monoclonal sécrété par la lignée cellulaire d'hybridome RF-HBs-1 (C.N.C.M. I-117) aux fins d'utilisation comme agent anti-viral contre le virus de l'hepatite B.